(19) Europäisches Patentamt
European Patent Office
Office européen des brevets

(11) **EP 2 172 172 A1**

(12) **EUROPEAN PATENT APPLICATION**

(43) Date of publication:
**07.04.2010 Bulletin 2010/14**

(51) Int Cl.:
***A61F 13/15*** (2006.01)    ***A61L 15/34*** (2006.01)
***A61L 15/46*** (2006.01)

(21) Application number: **09252235.8**

(22) Date of filing: **21.09.2009**

| | |
|---|---|
| (84) Designated Contracting States:<br>**AT BE BG CH CY CZ DE DK EE ES FI FR GB GR HR HU IE IS IT LI LT LU LV MC MK MT NL NO PL PT RO SE SI SK SM TR**<br>Designated Extension States:<br>**AL BA RS**<br><br>(30) Priority: **22.09.2008 US 99010 P**<br><br>(71) Applicant: **McNeil-PPC, Inc.**<br>**Skillman, NJ 08558 (US)**<br><br>(72) Inventors:<br>• **Burrow, Ricky R**<br>**Doylestown, PA 18901 (US)**<br>• **Luizzi, Joseph M.**<br>**Newtown, PA 18940 (US)** | • **Mavinkurve, Pramod**<br>**Princeton, NJ 08540 (US)**<br>• **Michaels, Elaine**<br>**Hoboken, NJ 07030 (US)**<br>• **Moscherosch, H. Michael**<br>**Doylestown, PA 19801 (US)**<br>• **Pataia, Vittorio**<br>**78100 Saint Germain en Laye (FR)**<br>• **Begay, Jessica**<br>**Lakewood, CO 80228 (US)**<br><br>(74) Representative: **Kirsch, Susan Edith et al**<br>**Carpmaels & Ransford**<br>**43-45 Bloomsbury Square**<br>**London WC1A 2RA (GB)** |

(54) **Absorbent article including fragrance emitting layer**

(57)    A sanitary absorbent article including a cover layer, a barrier layer, a secondary barrier layer arranged between the cover layer and the barrier layer, the secondary barrier layer provided with an oil based fragrance, wherein the secondary barrier layer is a mineral oil polymer blend microporous film.

Fig. 1

**Description**

## FIELD OF THE INVENTION

The present invention relates to an absorbent article including a fragrance emitting layer, and more particularly to a feminine hygiene article such as a sanitary napkin or liner that includes a fragrance emitting microporous film layer.

## BACKGROUND OF THE INVENTION

Sanitary protection articles, such as sanitary napkins and liners, that include a fragrance are well known in the art. Fragrances have been incorporated into these articles in the past to mask undesirable odors and such fragrances can also be functionalized with an odor-controlling agent. Sanitary napkins and liners disclosed in the art generally include a liquid permeable cover layer and a liquid impermeable barrier layer. Such articles may further include a core layer arranged between the cover and barrier layers, and may optionally include a transfer layer arranged between the cover layer and the core. Often the constituent layers of the absorbent article are secured to one another by means of a construction adhesive. Also, the absorbent article typically includes a positioning adhesive arranged on a garment facing surface of the article for securing the article to the undergarment during use.

The inventors have discovered that many fragrances used in absorbent articles will migrate into the adhesive components of the article and undesirably interact with the adhesive by altering its chemical composition. In particular, the inventors have discovered the aromatic components of many fragrances tend to plasticize the end blocks of standard hot melt adhesives. The inventors have discovered that this interaction causes the adhesives to perform poorly by reducing the cohesiveness and internal strength of standard construction and positioning adhesives. Specifically, the inventors have discovered that the interaction between the fragrance and positioning adhesive may cause the article to detach from the undergarment and/or may interact with the construction adhesive thereby causing the undesirable delamination of the layers of the article.

The inventors have further discovered that in order for an absorbent article to provide the desired intensity of scent, and in order for the scent to last for a sufficient period of time during use, the fragrance must be applied to the relevant layer of the article in a relatively high add on amount. However, the inventors have discovered that the use of a high add on amount of fragrance exacerbates the degradation of the adhesive described above.

In view of the foregoing, the present invention provides an absorbent article construction that has the ability to incorporate high levels of fragrance without sacrificing the functionality of the construction and positioning adhesives.

## SUMMARY OF THE INVENTION

In view of the foregoing, the present invention provides a sanitary absorbent article including a cover layer, a barrier layer, a secondary barrier layer arranged between the cover layer and the barrier layer, the secondary barrier layer provided with an oil based fragrance, wherein the secondary barrier layer is a mineral oil polymer blend microporous film.

## BRIEF DESCRIPTION OF THE DRAWINGS

FIG. 1 is a plan view of a sanitary absorbent article according to the present invention; and

FIG. 2 is an exploded view of a the sanitary napkin shown in FIG. 1.

## DETAILED DESCRIPTION OF THE INVENTION

The present invention will be described herein with reference to a sanitary napkin, however the present invention is equally applicable to other sanitary absorbent articles such as panty liners and adult incontinence products.

As used herein, the term "construction adhesive" refers to any adhesive that is used to join two layers of material to one another.

As used herein, the term "positioning adhesive" refers to any adhesive that is used to selectively attach a sanitary absorbent article to an undergarment.

The present invention relates to a sanitary absorbent article, such as a sanitary napkin, that includes a fragrance to thereby provide a fresh scent and/or mask odor. The inventors have found that in order to achieve long lasting fragrance levels, a high amount of fragrance must be incorporated into the absorbent article. The inventors have found that the fragrance should preferably be applied in an add on amount of greater than 3 gsm ($g/m^2$). Suitably, the fragrance is applied to at least one of the layers of the article in an amount of between about 3 gsm and about 15 gsm.

The inventors have discovered that purposely selecting fragrances that are substantially insoluable in the adhesive compounds used in the absorbent article minimizes the undesireable reaction between these components. The Hildebrand solubility parameter is used often in chemistry to predict when two solutions are soluble in one another. According to the theory proposed by Dr. Joel Hildebrand, two solutions will be soluble when the Hildebrand solubility parameter is equal, and insoluble when the Hildebrand solubility parameter is not equal. The difference between the two values is roughly related to the extent of insolubility between the two solutions. The Hildebrand solubility parameter ($\delta(SI)$) is derived from the heat of vaporization ($\Delta H$), the universal gas constant (R), the temperature (T), and the molar volume of the solution ($V_m$), and is calculated using the following formula:

$$\delta(SI) = [(\Delta H - RT) / V_m]^{1/2}$$

The resulting value is a property of a particular solution at a given temperature. In the international system of units (SI), the universal gas constant (R) is approximately 8.314 $J \cdot K^{-1} \cdot mol^{-1}$. The Hildebrand solubility parameter has the units of $MPa^{1/2}$.

The Hildebrand solubility parameter of common adhesives and fragrances is provided in TABLE 1 below:

**TABLE 1**

| Classification | Solution | $\delta(SI)$ |
|---|---|---|
| Common Adhesives | Sytrenic Block Copolymers and Tackifying Resins | 14.4-18.6 |
| | Polyethylene, EVA | 17-18.6 |
| | Polypropylene Polymers | 17.2-19.2 |
| Common Fragrances | Pine Oil | 17.6 |
| | d-Limonene | 16.5 |
| | Vanillin | 24.7 |
| | Eugenol | 22.2 |
| | Citral | 18.7 |
| | Carvone | 18.7 |
| | Jasmone | 18.4 |

According to the present invention, the adhesive(s) and fragrance(s) employed in the absorbent article according to the present invention preferably have a solubility parameter absolute value difference of greater than 1.5, more preferably greater than 3.0, and most preferably greater than 5.0. This relationship can be expressed by the follow equation:

$$| \delta_a - \delta_f | > 1.5;$$

where
$\delta_a$ = Hildebrand solubility parameter of the adhesive, and
$\delta_f$ = Hildebrand solubility parameter of the fragrance.
Selection of a fragrance(s) and an adhesive(s) that satisfy the above equation insures that fragrance does not adversely interact with the ahesive and thereby compromise the same. This insures that, even at high fragrance add on levels, the absorbent will securly adhere to the undergarment during use and will not delaminate.

## DESCRIPTION OF THE PREFERRED EMBODIMENTS

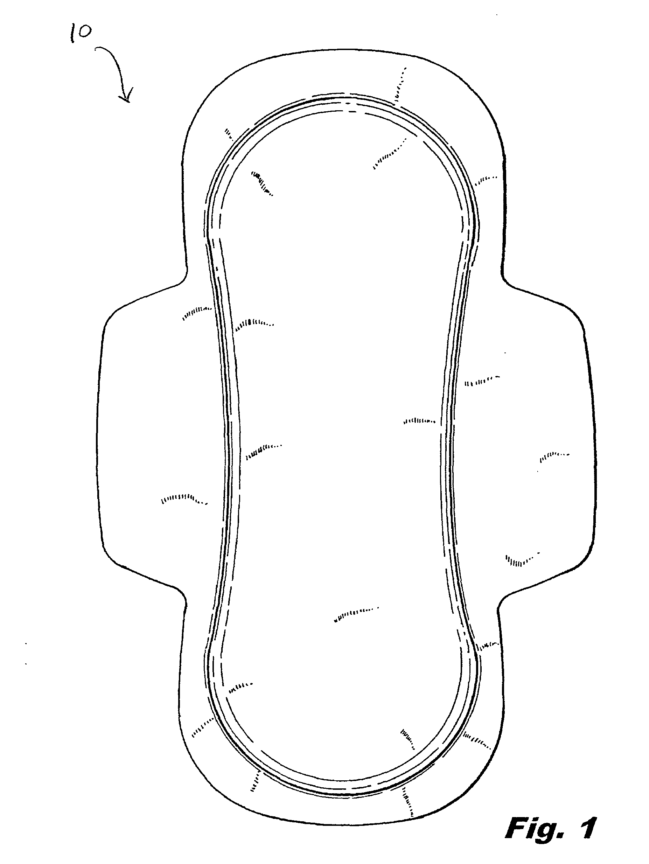

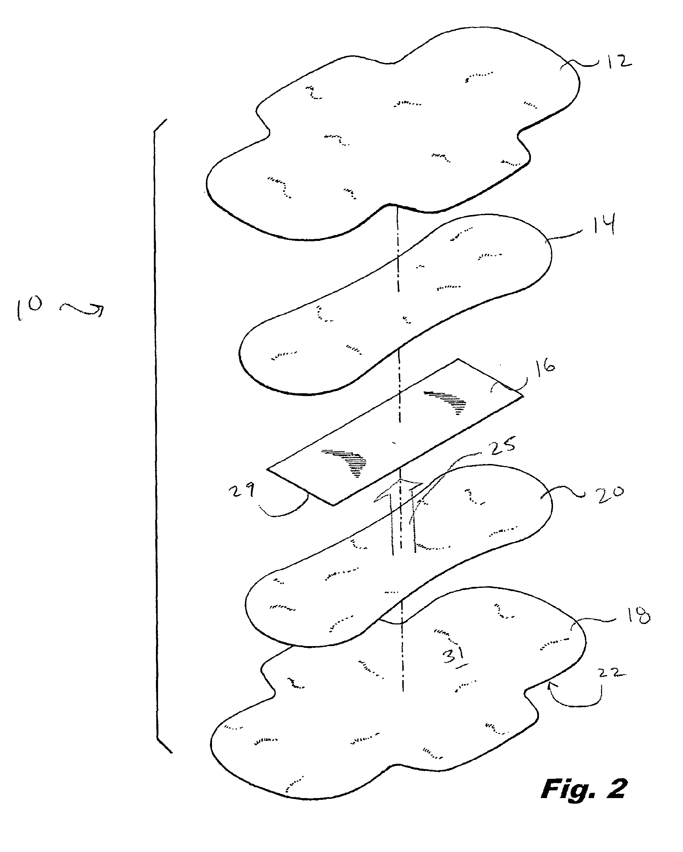

Fig. 1 and 2 illustrates an example of a sanitary napkin 10 according to the present invention. As shown in Fig.

2, the sanitary napkin 10 generally includes a liquid permeable body facing cover layer 12, an optional transfer layer 14, a core 16, a liquid impermeable barrier layer 18, and a secondary barrier layer 20. As shown in Fig. 2, the secondary barrier layer 20 is preferably arranged between a bottom surface 29 of the core 16 and a top surface 31 of the barrier layer 18.

A garment facing surface 22 of the barrier layer 18 may be provided with a positioning adhesive for securing the sanitary napkin to an undergarment during use. Suitable positioning adhesive compositions include hot melt adhesives based on block copolymers such as linear or radial co-polymer structures having the formula (A-B)$_x$ wherein block A is a polyvinylarene block, block B is a poly(monoalkenyl) block, and x is an integer greater than or equal to one that denotes the number of polymeric arms. Suitable block A polyvinylarenes include, but are not limited to, polystyrene, polyalpha-methylstyrene, polyvinyltoluene, and combinations thereof. Likewise, suitable Block B poly(monoalkenyl) blocks include, but are not limited to, conjugated diene elastomers, such as polybutadiene, polyisoprene, and hydrogenated elastomers such as ethylene butylenes, ethylene propylene, polyisobutylene, or combinations thereof. Commercial examples of these types of block copolymers include KratonTM elastomers from Kraton Polymers L.P, VectorTM elastomers from Dexco, SIBSTAR polymers from Kaneka USA, and StereonTM from Firestone Tire & Rubber Co. Alternately, suitable acrylic hot melt adhesive polymers such as the ACResin hot melt adhesives from BASF Corp. may also be used. In addition to providing some level of insolubility to the fragrances, these systems can be rendered further insoluble via crosslinking using a UV radiation source.

The sanitary napkin 10 may be optionally provided with a removable backing layer that is intended to protect the positioning adhesive prior to use of the sanitary napkin 10. The backing layer may be constructed of a suitable paper and/or polymeric film material. The surface of the backing layer in contact with the positioning adhesive may be provided with a non-stick coating such as silicone to facilitate the removal of the backing layer by the user prior to use.

According to one aspect of the invention, the secondary barrier layer 20 is provided with a fragrance 25. The fragrance 25 may be selected from one of the fragrances set forth in Table 1 above or may be selected from other common fragrances known to those of skill in the art. The fragrance 25 may also constitute a complex fragrance, i.e. a fragrance including a mixture of a number of different fragrance components. Typically the solubility parameter $\delta_f$ of such complex fragrance mixtures may be obtained from the commercial manufacturer of such fragrances. The fragrance 25 is preferably applied onto the secondary barrier layer 20 in an amount greater than about 3 gsm (g/m$^2$), preferably between about 3 gsm and about 15 gsm.

According to the present invention, the positioning adhesive and the fragrance 25 should preferably be selected such that they have a solubility parameter absolute value difference of greater than 1.5, more preferably greater than 3.0 and most preferably greater than 5.0. This relationship can be expressed by the follow equation:

$$| \delta_{pa} - \delta_f | > 1.5;$$

where
$\delta_{pa}$= Hildebrand solubility parameter of the positioing adhesive, and
$\delta_f$= Hildebrand solubility parameter of the fragrance.
Selection of a fragrance 25 and a positioining adhesive that satisfy the above equation insures that fragrance 25 does not adversely interact with the positioning ahesive and thereby compromise the same. This insures that, even at high fragrance add on levels, the absorbent article will remain securely attached to the undergarment during use.

The various layers of the sanitary napkin may be secured to one another by means of a construction adhesive arranged between the layers of the article. The construction adhesive is preferably selected from the same group of adhesives as the positioing adhesive. Thus suitable construction adhesive compositions include hot melt adhesives based on block copolymers such as linear or radial co-polymer structures having the formula (A-B)$_x$ wherein block A is a polyvinylarene block, block B is a poly(monoalkenyl) block, and x is an integer greater than or equal to one that denotes the number of polymeric arms. Suitable block A polyvinylarenes include, but are not limited to, polystyrene, polyalpha-methylstyrene, polyvinyltoluene, and combinations thereof. Likewise, suitable Block B poly(monoalkenyl) blocks include, but are not limited to, conjugated diene elastomers, such as polybutadiene, polyisoprene, and hydrogenated elastomers such as ethylene butylenes, ethylene propylene, polyisobutylene, or combinations thereof. Commercial examples of these types of block copolymers include KratonTM elastomers from Kraton Polymers L.P, VectorTM elastomers from Dexco, SIBSTAR polymers from Kaneka USA, and StereonTM from Firestone Tire & Rubber Co. Alternately, suitable acrylic hot melt adhesive polymers such as the ACResin hot melt adhesives from BASF Corp. may also be used. In addition to providing some level of insolubility to the fragrances, these systems can be rendered further insoluble via crosslinking using a UV radiation source.

According to the present invention, the construction adhesive and the fragrance 25 should preferably be selected such that they have a solubility parameter absolute value difference of greater than 1.5, more preferably greater than 3.0 and most preferably greater than 5.0. This relationship can be expressed by the follow equation:

$$| \delta_{ca} - \delta_f | > 1.5;$$

where

$\delta_{ca}$ = Hildebrand solubility parameter of the construction adhesive, and

$\delta_f$ = Hildebrand solubility parameter of the fragrance.

Selection of a fragrance 25 and a construction adhesive that satisfy the above equation insures that fragrance 25 does not adversely interact with the construction adhesive and thereby compromise the same. This insures that, even at high fragrance add on levels, the layers of the sanitary napkin 10 will remain secured adhered to one another and will not delaminate.

Cover Layer

The cover layer 12 may be a relatively low density, bulky, high-loft non-woven web material. The cover layer 12 may be composed of only one type of fiber, such as polyester or polypropylene or it may include a mixture of more than one fiber. The cover may be composed of bi-component or conjugate fibers having a low melting point component and a high melting point component. The fibers may be selected from a variety of natural and synthetic materials such as nylon, polyester, rayon (in combination with other fibers), cotton, acrylic fiber and the like and combinations thereof. Preferably, the cover layer 12 has a basis weight in the range of about 10 gsm to about 75 gsm.

Bi-component fibers may be made up of a polyester layer and a polyethylene sheath. The use of appropriate bi-component materials results in a fusible non-woven fabric. Examples of such fusible fabrics are described in U.S. Pat. No. 4,555,430 issued Nov. 26, 1985 to Chicopee. Using a fusible fabric increases the ease with which the cover layer may be mounted to the absorbent layer and/or to the barrier layer.

The cover layer 12 preferably has a relatively high degree of wettability, although the individual fibers comprising the cover may not be particularly hydrophilic. The cover material should also contain a great number of relatively large pores. This is because the cover layer 12 is intended to take-up body fluid rapidly and transport it away from the body and the point of deposition. Therefore, the cover layer contributes little to the time taken for the napkin to absorb a given quantity of liquid (penetration time).

Advantageously, the fibers which make up the cover layer 12 should not lose their physical properties when they are wetted, in other words they should not collapse or lose their resiliency when subjected to water or body fluid. The cover layer 12 may be treated to allow fluid to pass through it readily. The cover layer 12 also functions to transfer the fluid quickly to the underlying layers of the napkin. Thus, the cover layer 12 is advantageously wettable, hydrophilic and porous. When composed of synthetic hydrophobic fibers such as polyester or bi-component fibers, the cover layer 12 may be treated with a surfactant to impart the desired degree of wettability.

Alternatively, the cover layer 12 can also be made of polymer film having large pores. Because of such high porosity, the film accomplishes the function of quickly transferring body fluid to the inner layers of the underlying absorbent layers. A suitable cover material of this type is commercially found on the STAYFREE Dry Max Ultrathin product distributed by McNeil-PPC, Inc.

The cover layer 12 may be attached to the underlying absorbent layers 14 and 16, and/or the barrier layer 18, by adhesion and/or other suitable means know to those of skill in the art.

Transfer Layer

The transfer layer, 14 may be composed of fibrous materials, such as wood pulp, polyester, rayon, flexible foam, or the like, or combinations thereof. The transfer layer 14 may also optionally include a superabsorbent polymer (SAP) material. The transfer layer 14 may also comprise thermoplastic fibers for the purpose of stabilizing the layer and maintaining its structural integrity. The transfer layer 14 may be treated with surfactant on one or both sides in order to increase its wettability, although generally the transfer layer 14 is relatively hydrophilic and may not require treatment. The transfer layer 14 is preferably bonded on both sides to the adjacent layers, i.e. the cover layer 12 and the underlying core 16.

In one specific embodiment of the invention the transfer layer 14 is a through air bonded pulp material commercially available from Buckeye Technologies, Memphis, Tenn. under the designation VIZORB 3045.

Absorbent Core

The absorbent core 16 may comprise a single layer of material or may comprise multiple layers. In one embodiment, the core 16 is a blend or mixture of cellulosic fibers and superabsorbent disposed therein. Cellulosic fibers that can be used in the second absorbent layer 16 are well known in the art and include wood pulp, cotton, flax and peat moss. Wood pulp is preferred. Pulps can be obtained from mechanical or chemi-mechanical, sulfite, kraft, pulping reject materials, organic solvent pulps, etc. Both softwood and hardwood species are useful. Softwood pulps are preferred. It is not necessary to treat cellulosic fibers with chemical debonding agents, cross-linking agents and the like for use in the present material. Some portion of the pulp may be chemically treated as discussed in U.S. Pat. No. 5,916,670 to improved flexibility of the product. Flexibility of the material may also be improved by mechanically working the material or tenderizing the material.

The core 16 can contain any superabsorbent polymer (SAP) which are well known in the art. For the purposes of the present invention, the term "superabsorbent polymer" (or "SAP") refers to materials which are capable of absorbing and retaining at least about 10 times their weight in body fluids under a 0.5 psi pressure. The superabsorbent polymer particles of the invention may be inorganic or organic crosslinked hydrophilic polymers, such as polyvinyl alcohols, polyethylene oxides, crosslinked starches, guar gum, xanthan gum, and the like. The particles may be in the form of a powder, grains, granules, or fibers. Preferred superabsorbent polymer particles for use in the present invention are crosslinked polyacrylates, such as the product offered by Sumitomo Seika Chemicals Co., Ltd. Of Osaka, Japan, under the designation of SA70N and products offered by Stockhausen Inc. In a specific example, the core 16 is a material containing from 90% to about 40% percent cellulosic fiber, about 10% to about 60% SAP. The core 16 may comprise a material manufactured by using air-laying means well known in the art.

In one preferred embodiment of the invention the core 16 is relatively thin, high swelling absorbent material such as the absorbent composite material sold under the trade name NOVATHIN ® available from EAM Corporation located in Jessup, Ga. , U.S.A.

Barrier Layer

Underlying the secondary barrier 20, is a barrier layer 18 comprising liquid-impervious film material so as to prevent liquid that is entrapped in the absorbent layer 16 from egressing the sanitary napkin and staining the wearer's undergarment. The barrier layer 18 is preferably made of polymeric film, although it may be made of liquid impervious, air-permeable material such as repellent-treated non-woven or micropore films or foams.

The barrier layer 18 may be breathable, i.e., permits vapor to transpire. Known materials for this purpose include nonwoven materials and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet. The cover layer 12 and the barrier layer 18 are preferably joined along their marginal portions so as to form an enclosure or flange seal that maintains the absorbent layers 14 and 16 captive. The joint may be made by means of adhesives, heat-bonding, ultrasonic bonding, radio frequency sealing, mechanical crimping, and the like and combinations thereof.

Absorbent articles of this invention may or may not include wings, flaps or tabs for securing the absorbent article to an undergarment. Wings, also called, among other things, flaps or tabs, and their use in sanitary protection articles is described in U.S. Patent. No. 4,687,478 to Van Tilburg; U.S. Patent No. 4,589,876 also to Van Tilburg, U.S. Patent No. 4,900,320 to McCoy, and U.S. Patent No. 4,608,047 to Mattingly. The disclosures of these patents are incorporated herein by reference in their entirety. As disclosed in the above documents, wings are generally speaking flexible and configured to be folded over the edges of the underwear so that the wings are disposed between the edges of the underwear.

Secondary Barrier Layer

As shown in Fig. 2, the seconary barrier layer 20 is preferably arranged between a bottom surface 29 of the core 16 and a top surface 31 of the barrier layer 18.

Preferably, the seconary barrier layer 20 is a formed from a microporous film material, and more specifically a microporous film material made by a method including the steps of melt blending crystallizable thermoplastic polymer with a compound which is miscible with the thermoplastic polymer at the melting temperature of the polymer but phase seperates on cooling at or below the crystallization temperature of the polymer. Microporous film materials of this type are described in U.S. Patent No. 4,539,256. As described in US453926, microporous fims of this type are typically formed by blending a mineral oil with the polymer at an elevated temperature and then cooling the mixture to form a film. The film is then elongated to thereby form the microporous structure of the film. For purposes of simplicity, microporous films of this type will be refered to herein as "mineral oil polymer blend microporous films". Microporous films of this type are commercially available from Minnesota Mining and Manufacturing, Saint Paul, Minnesota. Microporous films of this type are particuarly useful for use in the present invention in that they effectively retain an oil based fragrance when such a fragrance is applied to the layer but at the same time permit the slow volitile release of the fragrance.

While particular embodiments of the present invention have been illustrated and described, it would be obvious to those skilled in the art that various other changes and modifications can be made without departing from the spirit and scope of the invention. It is cover in the appended claims all such changes and modifications that are within the scope of the invention.

## Claims

1.  A sanitary absorbent article comprising:

    a cover layer;
    a barrier layer;
    a secondary barrier layer arranged between the cover layer and the barrier layer, the secondary barrier layer provided with an oil based fragrance;

    wherein the secondary barrier layer is a mineral oil polymer blend microporous film.

2.  The sanitary absorbent article according to claim 1, further comprising a positioning adhesive arranged on a garment facing surface of the barrier layer.

3.  The sanitary absorbent article according to claim 1 or claim 2, wherein the secondary barrier layer is provided with the fragrance in an amount greater than about 3 gsm.

4.  The sanitary absorbent article according to claim 3, wherein the secondary barrier layer is provided with the fragrance in an amount within the range of about 3 gsm and about 15 gsm.

5.  The sanitary absorbent article according to claim 2, wherein an absolute difference of a Hildebrand solubility parameter of the positioning adhesive and a Hildebrand solubility parameter of the fragrance is greater than 1.5.

6.  The sanitary absorbent article according to any preceding claim, wherein one of the cover layer, barrier layer and secondary barrier layer is secured to another one of the cover layer, barrier layer and secondary barrier layer by means of a construction adhesive.

7.  The sanitary absorbent article according to claim 6, wherein an absolute difference of a Hildebrand solubility parameter of the construction adhesive and the Hildebrand solubility parameter of the fragrance is greater than 1.5.

8.  The sanitary absorbent article according to any preceding claim, further comprising an absorbent core arranged between the secondary barrier layer and the cover layer.

9.  The sanitary absorbent article according to claim 8, further comprising a transfer layer arranged between the cover layer and the absorbent core.

10. The sanitary absorbent article according to any preceding claim, wherein the absorbent article is one of a sanitary napkin, panty liner and adult incontinence product.

*Fig. 1*

Fig. 2

Europäisches
Patentamt

European
Patent Office

Office européen
des brevets

# EUROPEAN SEARCH REPORT

**Application Number**

EP 09 25 2235

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 713 069 A (WANG KENNETH Y [US] ET AL) 15 December 1987 (1987-12-15) | 1,8,10 | INV. A61F13/15 |
| Y | * figures 1,2 * | 2,5-7,9 | A61L15/34 |
| A | * column 8, line 49 - column 8, line 60 * | 3-4 | A61L15/46 |
| | * column 13, line 60 - column 13, line 65 * | | |
| | * column 14, line 4 - column 14, line 11 * | | |
| | ----- | | |
| Y | US 5 242 726 A (PARISEAU TIMOTHY P [US] ET AL) 7 September 1993 (1993-09-07) * column 1, line 6 - column 1, line 23 * * column 2, line 3 - column 2, line 11 * * column 4, line 14 - column 4, line 23 * ----- | 1-10 | |
| Y | EP 0 319 222 A2 (MINNESOTA MINING & MFG [US]) 7 June 1989 (1989-06-07) * page 3, line 36 - page 3, line 48 * * page 4, line 15 - page 4, line 26 * ----- | 1-10 | |
| Y | WO 94/06387 A1 (MINNESOTA MINING & MFG [US]; BOYER CHARLES E III [US]; KINNEY ROBERT J) 31 March 1994 (1994-03-31) * page 1, line 17 - page 1, line 22 * * examples 1-27 * ----- | 1-10 | TECHNICAL FIELDS SEARCHED (IPC) A61F A61L |
| Y | US 2002/004350 A1 (MORMAN MICHAEL TOD [US] ET AL) 10 January 2002 (2002-01-10) * paragraphs [0002], [0007], [0024], [0048], [0049] * ----- | 1-10 | |
| Y | US 5 849 001 A (TORIMAE YASUHIRO [JP] ET AL) 15 December 1998 (1998-12-15) * column 3, line 19 - column 3, line 57 * * column 6, line 21 - column 6, line 31 * ----- | 1-10 | |

The present search report has been drawn up for all claims

| Place of search | Date of completion of the search | Examiner |
|---|---|---|
| Munich | 14 January 2010 | Gennari, Silvia |

EPO FORM 1503 03.82 (P04C01)

## ANNEX TO THE EUROPEAN SEARCH REPORT
## ON EUROPEAN PATENT APPLICATION NO.

EP 09 25 2235

This annex lists the patent family members relating to the patent documents cited in the above-mentioned European search report.
The members are as contained in the European Patent Office EDP file on
The European Patent Office is in no way liable for these particulars which are merely given for the purpose of information.

14-01-2010

| Patent document cited in search report | | Publication date | Patent family member(s) | | Publication date |
|---|---|---|---|---|---|
| US 4713069 | A | 15-12-1987 | NONE | | |
| US 5242726 | A | 07-09-1993 | CA | 2108658 A1 | 24-03-1993 |
| | | | DE | 69216630 D1 | 20-02-1997 |
| | | | DE | 69216630 T2 | 24-04-1997 |
| | | | EP | 0605451 A1 | 13-07-1994 |
| | | | ES | 2096100 T3 | 01-03-1997 |
| | | | JP | 6511025 T | 08-12-1994 |
| | | | MX | 9204882 A1 | 01-04-1993 |
| | | | WO | 9306182 A1 | 01-04-1993 |
| EP 0319222 | A2 | 07-06-1989 | AR | 240241 A1 | 30-03-1990 |
| | | | AU | 2588888 A | 15-06-1989 |
| | | | BR | 8806398 A | 22-08-1989 |
| | | | JP | 2000504 A | 05-01-1990 |
| | | | JP | 7010580 B | 08-02-1995 |
| | | | US | 4824718 A | 25-04-1989 |
| | | | ZA | 8808910 A | 25-07-1990 |
| WO 9406387 | A1 | 31-03-1994 | AU | 663894 B2 | 26-10-1995 |
| | | | AU | 2785092 A | 12-04-1994 |
| | | | AU | 677912 B2 | 08-05-1997 |
| | | | AU | 3033995 A | 23-11-1995 |
| | | | BR | 9207147 A | 12-12-1995 |
| | | | CA | 2125481 A1 | 31-03-1994 |
| | | | CA | 2221172 A1 | 31-03-1994 |
| | | | CZ | 9500584 A3 | 14-06-1995 |
| | | | DE | 69229641 D1 | 26-08-1999 |
| | | | ES | 2133330 T3 | 16-09-1999 |
| | | | IL | 106812 A | 15-04-1997 |
| | | | JP | 2898756 B2 | 02-06-1999 |
| | | | JP | 8501458 T | 20-02-1996 |
| | | | ZA | 9306070 A | 20-02-1995 |
| US 2002004350 | A1 | 10-01-2002 | AR | 028390 A1 | 07-05-2003 |
| | | | AU | 5910301 A | 12-11-2001 |
| | | | AU | 2001259103 B2 | 19-05-2005 |
| | | | BR | 0110181 A | 05-03-2003 |
| | | | CN | 1427863 A | 02-07-2003 |
| | | | DE | 60120407 T2 | 19-10-2006 |
| | | | EP | 1299460 A1 | 09-04-2003 |
| | | | JP | 2003531937 T | 28-10-2003 |
| | | | MX | PA02010138 A | 10-03-2003 |
| | | | WO | 0183599 A1 | 08-11-2001 |
| | | | US | 2004091752 A1 | 13-05-2004 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 09 25 2235

This annex lists the patent family members relating to the patent documents cited in  the above-mentioned European search report.
The members are as contained in the European Patent Office EDP file on
The European Patent Office is in no way liable for these particulars which are merely given for the purpose of information.

14-01-2010

| Patent document cited in search report | | Publication date | Patent family member(s) | | Publication date |
|---|---|---|---|---|---|
| US 5849001 | A | 15-12-1998 | CN | 1136425 A | 27-11-1996 |
| | | | JP | 8269220 A | 15-10-1996 |

EPO FORM P0459

For more details about this annex : see Official Journal of the European Patent Office, No. 12/82

**REFERENCES CITED IN THE DESCRIPTION**

**Patent documents cited in the description**

- US 4555430 A, Chicopee
- US 5916670 A
- US 4687478 A, Van Tilburg
- US 4589876 A, Van Tilburg
- US 4900320 A, McCoy
- US 4608047 A, Mattingly
- US 4539256 A
- US 453926 A